(19) Europäisches Patentamt
European Patent Office
Office européen des brevets

(11) EP 1 136 123 B1

(12) FASCICULE DE BREVET EUROPEEN

(45) Date de publication et mention de la délivrance du brevet: **25.05.2005 Bulletin 2005/21**

(51) Int Cl.[7]: **B01J 31/22**, B01J 31/18, B01J 31/30, B01J 31/14, C07C 2/22, C07C 2/34

(21) Numéro de dépôt: **01400510.2**

(22) Date de dépôt: **28.02.2001**

# (54) Composition catalytique et procédé pour la catalyse de dimérisation, de codimérisation et d'oligomérisation des oléfines

Katalytische Zusammensetzung und katalytisches Verfahren zur Dimerisierung, Codimerisierung und Oligomerisierung von Olefinen

Catalytic composition and catalytic process of dimerisation, codimerisation and oligomerisation of olefins

(84) Etats contractants désignés:
**DE ES GB IT NL**

(30) Priorité: **23.03.2000 FR 0003819**

(43) Date de publication de la demande:
**26.09.2001 Bulletin 2001/39**

(73) Titulaire: **Institut Français du Pétrole
92852 Rueil-Malmaison Cedex (FR)**

(72) Inventeurs:
- **Olivier-Bourbigou, Hélène
92500 Rueil-Malmaison (FR)**
- **Commereuc, Dominique
92190 Meudon (FR)**
- **Harry, Stéphane
78360 Montesson (FR)**

(56) Documents cités:
**EP-A- 0 646 412**
**DE-A- 4 447 066**
**FR-A- 2 728 180**
**US-A- 5 324 799**
**EP-A- 0 798 041**
**FR-A- 2 611 700**
**US-A- 5 166 114**
**US-A- 5 728 839**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 136 123 B1

## Description

La présente invention concerne la dimérisation, la codimérisation et l'oligomérisation catalytique des oléfines.

Elle a pour objet une nouvelle composition catalytique, la codimérisation ou résultant de la dissolution d'au moins un complexe du nickel contenant au moins un ligand carbène hétérocyclique, dans le mélange liquide, à caractère ionique, d'au moins un halogénure d'ammonium et/ou de phosphonium quaternaire, d'au moins un trihalogénure d'aluminium et facultativement d'au moins un halogénure d'alkylaluminium. La présente invention a également pour objet l'utilisation de cette composition catalytique dans des procédés de dimérisation, de codimérisation et/ou d'oligomérisation des oléfines.

Certains complexes organométalliques du nickel contenant des ligands carbènes hétérocycliques ont été décrits dans l'art antérieur (demande internationale WO-A-99/6004, brevet US-A-5 728 839 et demande de brevet EP-A-0 798 041). La demande WO-A-99/6004 décrit également l'utilisation de ces complexes pour la polymérisation des acrylates et l'hydrocyanation des oléfines. De tels complexes ont l'avantage d'être très stables. Plus particulièrement, ces ligands monocarbènes ou bicarbènes conduisent à des complexes du nickel stables thermiquement et chimiquement surtout vis-à-vis de l'oxydation.

Cependant, certains de ces complexes du nickel présentent l'inconvénient d'être très peu solubles dans les solvants organiques classiques, ce qui en limite l'utilisation. La demande de brevet internationale WO-A-99/6004, décrit la préparation de complexes cationiques carbéniques du nickel solubles dans l'eau. Cependant, l'utilisation de l'eau ne peut pas se généraliser aux réactions dont l'espèce catalytique active met en jeu une liaison métal-carbone, qui se dégrade en présence de protons. C'est le cas de la dimérisation ou l'oligomérisation des oléfines.

Le brevet français FR-B-2 611 700 décrit l'utilisation de liquides à caractère ionique formés d'halogénures d'aluminium et d'halogénures d'ammonium quaternaires comme solvants de complexes organométalliques du nickel pour la catalyse de dimérisation des oléfines. L'utilisation de tels milieux non miscibles avec les hydrocarbures aliphatiques, en particulier avec les produits issus de la dimérisation des oléfines, permet une meilleure utilisation des catalyseurs homogènes. Le brevet français FR-B-2 659 871 décrit une composition liquide à caractère ionique résultant de la mise en contact d'au moins un halogénure d'ammonium quaternaire et/ou de phosphonium quaternaire avec au moins un dihalogénure d'alkylaluminium et éventuellement en outre au moins un trihalogénure d'aluminium. Ce brevet décrit également l'utilisation de ces milieux comme solvants de complexes de métaux de transition, notamment des complexes du nickel ne contenant pas de liaison nickel-carbone, qui sont transformés en catalyseurs d'oligomérisation des oléfines. Dans la suite, ces milieux seront appelés « sels fondus » parce qu'ils sont liquides à température modérée.

Au cours de ces travaux, il a été montré que les catalyseurs de nickel les plus actifs sont obtenus dans des « sels fondus » constitués d'un équivalent molaire d'halogénure d'ammonium quaternaire et/ou d'halogénure de phosphonium quaternaire avec un équivalent (ou plus) de trihalogénure d'aluminium et facultativement une quantité quelconque de dihalogénure d'alkyl-aluminium. Cette formulation s'est révélée particulièrement intéressante parce que les complexes du nickel qui y sont dissous présentent une activité catalytique élevée.

Les ligands neutres décrits dans ces travaux et facultativement associés au nickel sont des phosphines tertiaires. L'inconvénient des ligands alkylphosphines est que ce sont des composés onéreux et qui s'oxydent facilement en présence d'air.

Il a maintenant été trouvé que les complexes du nickel portant au moins un ligand monocarbène ou bicarbène répondant par exemple aux formules (I) et (II) données ci-après sont solubles et stables dans les milieux « sels fondus » et qu'ils permettent de catalyser la dimérisation, la codimérisation ou l'oligomérisation des oléfines.Ces ligands carbènes ont fait l'objet d'une revue dans Angew. Chem. Int. Ed. Engl. 1997, 36, 2162. Ce sont des ligands σ-donneurs et π-accepteurs qui forment avec les métaux de transition des liaisons très stables. Leurs propriétés électroniques peuvent être comparées à celles des trialkylphosphines basiques.

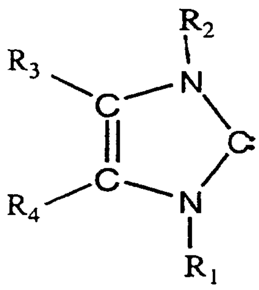
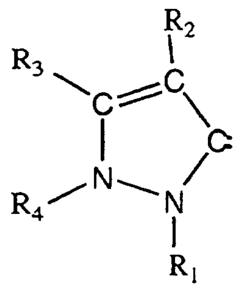
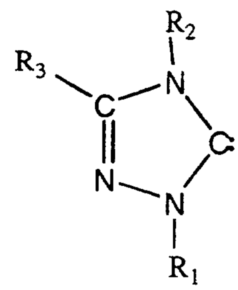

Type I

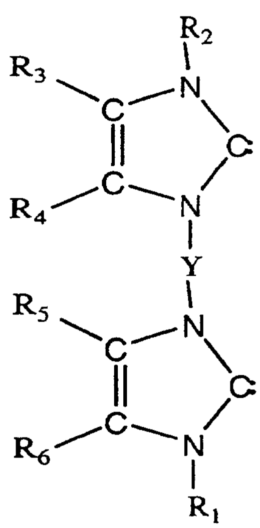
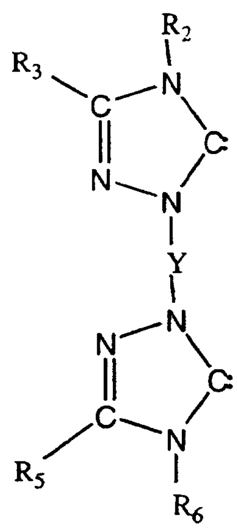
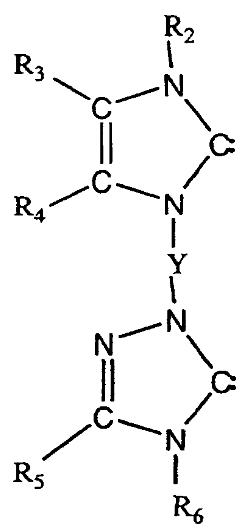

Type II

L'invention a donc pour objet une composition catalytique résultant de la dissolution d'un composé du nickel contenant au moins un ligand mono- ou bi- carbène hétérocyclique dans un milieu « sel fondu » constitué d'au moins un halogénure d'ammonium quaternaire et/ou au moins un halogénure de phosphonium quaternaire (Produit A), d'au moins un halogénure d'aluminium (Produit B) et facultativement d'au moins un composé organique de l'aluminium (Produit C).

Les composés du nickel utilisés selon l'invention sont des sels de nickel ou des composés organométalliques, chargés ou non, qui répondent à la formule générale (déjà décrite dans la demande de brevet EP-A-0 798 041) :

$$(Ni_aX_bY_dL_c)^n (A)_n$$

dans laquelle :

- a, b, c, d et n sont des nombres entiers avec a égal à 1, 2 ou 3 ; b de 0 à 2 fois a ; d de 0 à 2 fois a ; c de 1 à 4

fois b ; n égal à 0, 1 ou 2 ;

- X et Y, identiques ou différents, représentent chacun un ligand mono- ou multi-chélatant, chargé ou non ; à titre d'exemples, on pourra citer les halogénures, les carboxylates (par exemple l'éthyl-2-hexanoate), l'acétyl-acétonate, le sulfate, les phénates, les mono- et di-oléfines, les π-aromatiques, les radicaux alkyles ou aryles, les phosphines, les phosphites et le monoxyde de carbone ;
- L est un mono- ou bi-carbène hétérocyclique qui répond par exemple à l'une des formules générales (I) et (II) ci-dessus, dans lesquelles $R_1$, $R_2$, $R_3$, $R_4$, $R_5$ et $R_6$, identiques ou différents, représentent chacun l'hydrogène, un groupe hydrocarboné, aliphatique, saturé ou insaturé, ou aromatique, comprenant de 1 à 12 atomes de carbone, et Y représente un reste bivalent aliphatique de 1 à 4 atomes de carbone ; et
- A est un anion faiblement coordinant ; à titre d'exemples, on pourra citer les anions tétrafluoroborate, hexafluorophosphate, tétraphénylborate et leurs dérivés, les anions tétrachloroaluminate, hexafluoroantimonate, trifluoroacétate, trifluorométhylsulfonate et acétate.

Les carbènes hétérocycliques L peuvent être générés à partir des sels d'imidazolium ou bis(azolium) correspondants, par déprotonation. Le métal de transition peut jouer le rôle de réducteur.

A titre d'exemples non limitatifs de ligands mono- ou bicarbènes hétérocycliques, on citera les ligands carbènes décrits par les formules (1), (2) et (3) données ci-après.

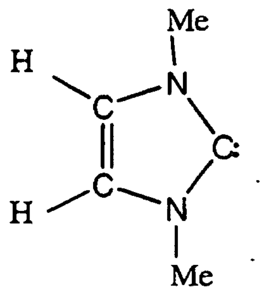

(1)

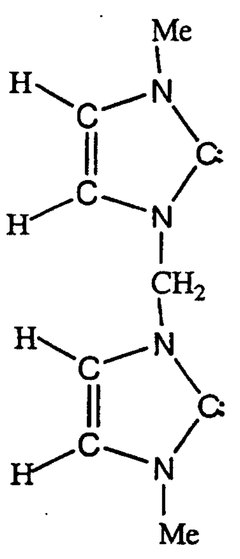

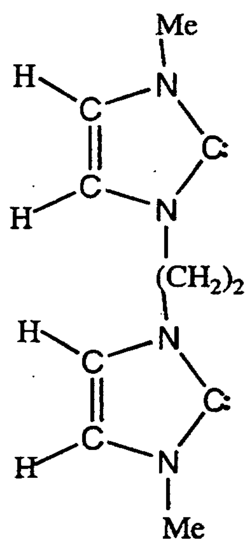

(2) (3)

A titre d'exemples non limitatifs de composés du nickel utilisables selon l'invention, on peut citer les complexes $NiCl_2$,[diméthyl-1,3-imidazolylidène-2]$_2$ ; $NiI_2$,[diméthyl-1,3-imidazolylidène-2]$_2$ ; le chlorure de π-allyl nickel (diméthyl-1,3-imidazolylidène-2) ; $NiI_2$,[diméthyl-1,1'-imidazol-diylidène-2,2'-méthylène-3,3']$_2$ et $NiI_2$,[diméthyl-1,1'-imidazol-diylidène-2,2'-éthylène-3,3']$_2$.

Les « sels fondus » mis en jeu selon l'invention sont constitués :

a) d'au moins un halogénure, plus particulièrement un chlorure et/ou un bromure, d'ammonium quaternaire et/ou de phosphonium quaternaire (A) ;
b) d'au moins le trichlorure d'aluminium et/ou le tribromure d'aluminium (B) ; et
c) facultativement d'un composé organique de l'aluminium (C).

Les halogénures d'ammonium et/ou de phosphonium quaternaires utilisables dans le cadre de l'invention (Produit A) répondent de préférence

- à l'une des formules générales $NR^1R^2R^3R^4X$ (à l'exception de $NH_4X$), $PR^1R^2R^3R^4X$, $R^1R^2N=CR^3R^4X$ ou $R^1R^2P=CR^3R^4X$, dans lesquelles X représente Cl ou Br et $R^1$, $R^2$, $R^3$ et $R^4$, identiques ou différents, représentent chacun l'hydrogène ou un reste hydrocarbyle de 1 à 12 atomes de carbone par exemple des groupements alkyles, saturés ou non saturés, cycloalkyles ou aromatiques, aryles ou aralkyles, comprenant de 1 à 12 atomes de carbone, étant entendu que, de préférence, un seul des substituants $R^1$, $R^2$, $R^3$ et $R^4$ représente l'hydrogène ;
- ou encore à l'une des formules générales :

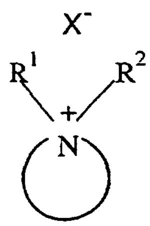 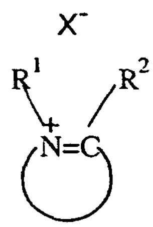 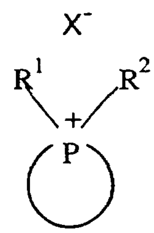 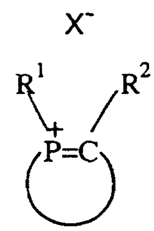

dans lesquelles les hétérocycles azotés ou phosphorés comportant 1, 2 ou 3 atomes d'azote et/ou de phosphore sont constitués de 4 à 10 atomes et X, $R^1$ et $R^2$ sont définis comme précédemment.

A titre d'exemples, on peut citer le chlorure de tétrabutylphosphonium, le chlorure de N-butylpyridinium, le bromure d'éthylpyridinium, le chlorure de butyl-3 méthyl-1 imidazolium, le chlorure de diéthylpyrazolium, le chlorhydrate de pyridinium, le chlorure de triméthylphénylammonium et le chlorure d'éthyl-3 méthyl-1 imidazolium. Ces sels peuvent être utilisés seuls ou en mélanges.

Les halogénures d'aluminium utilisés comme Produit B dans la composition des « sels fondus » selon l'invention sont essentiellement le chlorure et le bromure d'aluminium.

Les composés organiques de l'aluminium utilisés facultativement comme Produits C dans la composition des « sels fondus » selon l'invention ont pour formule générale $AlR_xX_{3-x}$, dans laquelle R est un reste hydrocarboné, par exemple alkyle, linéaire ou ramifié, comportant de 2 à 8 atomes de carbone, X étant le chlore ou le brome et x ayant une valeur égale à 1, 2 ou 3. A titre d'exemples, on peut utiliser le sesquichlorure d'isobutylaluminium, le sesquichlorure d'éthylaluminium, le dichloroisobutylaluminium, le dichloroéthylaluminium ou le chlorodiéthylaluminium.

Les constituants des « sels fondus » tels que définis ci-dessus sont en général mis en oeuvre dans des rapports molaires A:B de 1:0.5 à 1:3, de préférence de 1:1 à 1:2 ; le Produit C est mis en oeuvre dans un rapport molaire avec le Produit B au plus égal à 100:1 et de préférence de 0.005:1 à 10:1. Il est néanmoins nécessaire que les composants et leurs proportions soient tels que le mélange soit liquide à la température à laquelle se fait l'introduction du composé du nickel bien que la réaction catalytique de dimérisation, de codimérisation ou d'oligomérisation puisse se faire à une température inférieure ou supérieure à la température de fusion de la composition catalytique.

Les composés entrant dans la composition selon l'invention, peuvent être mélangés dans un ordre quelconque. Le mélange peut se faire par une simple mise en contact suivie d'une agitation jusqu'à formation d'un liquide homogène.

Les oléfines susceptibles d'être dimérisées, codimérisées ou oligomérisées par les compositions catalytiques selon l'invention sont l'éthylène, le propylène, les n-butènes et les n-pentènes, seuls ou en mélange, purs ou dilués par un ou plusieurs alcane(s), tels qu'on les trouve dans des « coupes » issues des procédés de raffinage du pétrole, comme le craquage catalytique ou le craquage à la vapeur.

La réaction catalytique de dimérisation, de codimérisation ou d'oligomérisation des oléfines peut être conduite en système fermé, en système semi-ouvert ou en continu avec un ou plusieurs étages de réaction. Une vigoureuse agitation doit assurer un bon contact entre le ou les réactifs et le mélange catalytique. La température de réaction peut être de -40 à +70 °C, de préférence de -20 à +50 °C. On peut opérer au-dessus ou en dessous de la température de fusion du milieu, l'état solide dispersé n'étant pas une limitation au bon déroulement de la réaction. La chaleur engendrée par la réaction peut être éliminée par tous les moyens connus de l'homme du métier. La pression peut être comprise entre la pression atmosphérique et 200 atmosphères (environ 20 MPa), de préférence entre la pression atmosphérique et 50 atmosphères (environ 5 MPa). Les produits de la réaction et le ou les réactifs qui n'ont pas réagi sont séparés du système catalytique par simple décantation, puis fractionnés.

Les exemples suivants illustrent l'invention sans en limiter la portée.

**EXEMPLE 1 :** Préparation du solvant ionique.

On a mélangé à température ambiante 17,5 g (0,1 mole) de chlorure de butylméthyl imidazolium, 16,3 g (0,122 mole) de trichlorure d'aluminium sublimé, 1,42 g (0,0112 mole) de dichloroéthylaluminium. On obtient une composition liquide.

**EXEMPLE 2 :** Préparation du complexe de nickel $NiI_2$,[diméthyl-1,3-imidazolylidène-2]$_2$ :

Le complexe de nickel est préparé par une amélioration de la synthèse décrite dans Organometallics, 1997, 16, 2209.

Dans un tube de type Schlenk, on agite fortement l'acétate de nickel préalablement séché (6 mmoles) avec l'iodure de diméthyl-1,3-imidazolium (12 mmoles) dans le nitrométhane (60 mL). On chauffe à 150 °C en tirant sous le vide de la pompe pendant une heure. On laisse refroidir puis on extrait le complexe rouge formé avec du tétrahydrofuranne chaud (500 mL). Le tétrahydrofuranne est ensuite évaporé sous vide et le composé rouge est lavé à l'éther diéthylique (140 mL). Un deuxième lavage avec de l'éthanol absolu (15 mL) est nécessaire pour éliminer l'iodure de diméthyl-1,3 imidazolium qui n'a pas réagi.

**EXEMPLE 3 :** Dimérisation du butène

Un réacteur en verre de 100 mL muni d'une sonde de mesure de température, d'un barreau aimanté pour assurer une bonne agitation et d'une double enveloppe permettant la circulation d'un liquide de réfrigération, a été purgé d'air et d'humidité, et maintenu à la pression atmosphérique de butène-1. On y a introduit 50,2 mg (0,1 mmole) du complexe $NiI_2$,[diméthyl-1,3-imidazolylidène-2]$_2$ obtenu à l'Exemple2, puis on a abaissé la température a 10 °C et injecté à l'aide d'une seringue 5 mL de la composition liquide obtenue à l'Exemple 1 On a mis l'agitation en route et on a observé immédiatement une absorption de butène. Quand le réacteur a été aux trois quarts plein de liquide, on a arrêté l'agitation, on a laissé se décanter le « sel fondu » et on a soutiré la plus grande partie de la phase hydrocarbonée. On a recommencé l'opération 5 fois. La durée totale de la réaction est de 5 heures et 25 minutes. A ce moment, on avait introduit au total 240 g de butène. On a produit 24 kg de produits par gramme de Ni. L'analyse des différentes fractions a montré qu'elles étaient composées à 94-96 % en poids de dimères.

## Revendications

**1.** Composition catalytique résultant de la dissolution d'au moins un composé du nickel contenant au moins un ligand mono- ou bi-carbène hétérocyclique dans un milieu constitué

- d'au moins un halogénure d'ammonium quaternaire et/ou au moins un halogénure de phosphonium quaternaire, désigné par Produit A ;
- d'au moins un halogénure d'aluminium, désigné par Produit B ;
- et facultativement d'au moins un composé organique de l'aluminium, désigné par Produit C.

**2.** Composition catalytique selon la revendication 1 dans laquelle ledit composé du nickel répond à la formule générale $(Ni_aX_bY_dL_c)^n (A)_n$ dans laquelle

- a, b, c, d et n sont des nombres entiers avec a égal à 1, 2 ou 3 ; b de 0 à 2 fois a ; d de 0 à 2 fois a ; c de 1 à 4 fois b ; et n égal à 0, 1 ou 2 ;
- X et Y, identiques ou différents, représentent chacun un ligand mono- ou multi-chélatant, chargé ou non ;
- L est un ligand mono- ou bi-carbène hétérocyclique ; et
- A est un anion faiblement coordinant.

**3.** Composition catalytique selon la revendication 2 **caractérisée en ce que** le ligand L répond à l'une des formules générales (I) et (II) de la description, dans lesquelles $R_1$, $R_2$, $R_3$, $R_4$, $R_5$ et $R_6$, identiques ou différents, représentent

chacun l'hydrogène, un groupe hydrocarboné, aliphatique, saturé ou insaturé, ou aromatique, comprenant de 1 à 12 atomes de carbone et Y représente un reste bivalent aliphatique de 1 à 4 atomes de carbone.

**4.** Composition catalytique selon la revendication 2 ou 3 **caractérisée en ce que** L est choisi parmi les ligands carbènes répondant aux formules (1), (2) et (3) de la description.

**5.** Composition catalytique selon l'une des revendications 2 à 4 **caractérisée en ce que** X et Y sont choisis parmi les halogénures, les carboxylates, l'acétyl-acétonate, le sulfate, les phénates, les mono- et di-oléfines, les $\pi$-aromatiques, les radicaux alkyles ou aryles, les phosphines, les phosphites et le monoxyde de carbone.

**6.** Composition catalytique selon l'une des revendications 2 à 5 **caractérisée en ce que** A est choisi parmi les anions tétrafluoroborate, hexafluorophosphate, tétraphénylborate et dérivés, tétrachloroaluminate, hexafluoroantimonate, trifluoroacétate, trifluorométhylsulfonate et acétate.

**7.** Composition catalytique selon les revendications 1 à 6 **caractérisée en ce que** le composé du nickel est choisi parmi

- $NiCl_2$,[diméthyl-1,3-imidazolylidène-2]$_2$ ;
- $NiI_2$,[diméthyl-1,3-imidazolylidène-2]$_2$ ;
- le chlorure de $\pi$- allyl nickel (diméthyl-1,3-imidazolylidène-2) ;
- $NiI_2$ [diméthyl-1,1'-imidazol-diylidène-2,2'-méthylène-3,3']$_2$ ; et
- $NiI_2$ [diméthyl-1,1'-imidazol-diylidène-2,2'-éthylène-3,3']$_2$.

**8.** Composition catalytique selon l'une des revendications 1 à 7 **caractérisée en ce que** l'halogénure d'ammonium quaternaire ou l'halogénure de phosphonium quaternaire utilisable comme Produit A répond

- à l'une des formules générales :
  $NR^1R^2R^3R^4X$, à l'exception de $NH_4X$, $PR^1R^2R^3R^4X$, $R^1R^2N{=}CR^3R^4X$ ou $R^1R^2P{=}CR^3R^4X$,
  dans lesquelles X représente Cl ou Br et $R^1$, $R^2$, $R^3$ et $R^4$, identiques ou différents, représentent chacun l'hydrogène ou un reste hydrocarbyle de 1 à 12 atomes de carbone ;
- ou à l'une des formules générales :

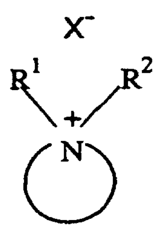
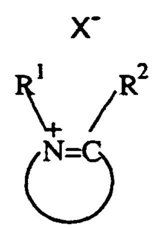
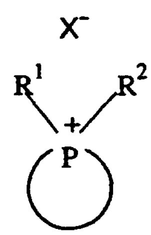
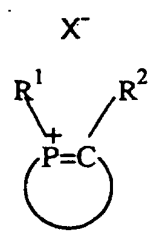

dans lesquelles les hétérocycles azotés ou phosphorés comportant 1, 2 ou 3 atomes d'azote et/ou de phosphore sont constitués de 4 à 10 atomes et X, R1 et R2 sont définis comme précédemment.

**9.** Composition catalytique selon la revendication 8 **caractérisée en ce que** l'halogénure d'ammonium quaternaire ou l'halogénure de phosphonium quaternaire est le chlorure de tétrabutylphosphonium, le chlorure de N-butylpyridinium, le bromure d'éthylpyridinium, le chlorure de butyl-3 méthyl-1 imidazolium, le chlorure de diéthylpyrazolium, le chlorhydrate de pyridinium, le chlorure de triméthylphénylammonium ou le chlorure d'éthyl-1 méthyl-3 imidazolium.

**10.** Composition catalytique selon l'une des revendications 1 à 9 **caractérisée en ce que** l'halogénure d'aluminium utilisé comme Produit B est le trichlorure ou le tribromure d'aluminium.

**11.** Composition catalytique selon l'une des revendications 1 à 10 **caractérisée en ce que** les Produits A et B sont mis en oeuvre dans un rapport molaire A:B de 1:0.5 à 1:3.

**12.** Composition catalytique selon l'une des revendications 1 à 11 **caractérisée en ce que** le milieu non aqueux à caractère ionique comprend en outre un Produit C, consistant en au moins un composé organique de l'aluminium.

**13.** Composition catalytique selon la revendication 12 **caractérisée en ce que** le composé organique de l'aluminium utilisé comme Produit C répond à la formule générale $AlR_xX_{3-x}$, dans laquelle R est un reste alkyle, linéaire ou ramifié, comportant de 2 à 8 atomes de carbone, X étant le chlore ou le brome et x a une valeur égale à 1, 2 ou 3.

**14.** Composition catalytique selon la revendication12 ou 13 **caractérisée en ce que** le produit C est le sesquichlorure d'isobutylaluminium, le sesquichlorure d'éthylaluminium, le dichloroisobutylaluminium, le dichloroéthylaluminium ou le chlorodiéthylaluminium.

**15.** Composition catalytique selon l'une des revendications 12 à 14 **caractérisée en ce que** le Produit C est mis en oeuvre dans un rapport molaire avec le Produit B au plus égal à 100:1

**16.** Procédé de dimérisation, de codimérisation ou d'oligomérisation d'au moins une oléfine, **caractérisé en ce que** ladite oléfine est mise en contact avec une composition catalytique selon l'une des revendications 1 à 15.

**17.** Procédé selon la revendication 16 **caractérisé en ce que** la réaction de dimérisation, de codimérisation ou d'oligomérisation des oléfines est conduite en système fermé, en système semi-ouvert ou en continu, avec un ou plusieurs étages de réaction, sous agitation et à une température de -40 à +70 °C.

**18.** Procédé selon l'une des revendications 16 et 17 **caractérisé en ce que** l'(les) oléfine(s) est (sont) choisie(s) parmi l'éthylène, le propylène, les n-butènes et les n-pentènes et leurs mélanges, purs ou dilués par au moins un alcane.

**19.** Procédé selon l'une des revendications 16 à 18 **caractérisé en ce que** l'(les) oléfine(s) est (sont) contenue(s) dans une « coupe » issue d'un procédé de raffinage du pétrole.

## Patentansprüche

**1.** Katalytische Zusammensetzung die aus dem Lösen wenigstens einer Nickelverbindung resultiert, die wenigstens einen Mono- oder Bicarbenliganden enthält, der heterozyklisch ist, in einem Medium, das besteht aus:

- wenigstens einem Halogenid eines quaternären Ammoniums und/oder wenigstens einem Halogenid eines quaternären Phosphoniums, bezeichnet als Produkt A;
- wenigstens ein Aluminiumhalogenid, bezeichnet als Produkt B;
- und fakultativ wenigstens eine organische Aluminiumverbindung, bezeichnet als Produkt C.

**2.** Katalytische Zusammensetzung nach Anspruch 1, bei der die Nickelverbindung der allgemeinen Formel $(Ni_aX_bY_dL_c)^n (A)_n$ entspricht, in welcher

- a b, c, d und n ganze Zahlen mit a gleich 1, 2 oder 3, b gleich 0 bis 2mal a; d gleich 0 bis 2mal a; c 1 bis 4mal b; und n gleich 0, 1 oder 2 sind;
- X und Y identisch oder verschieden jedes einen mono- oder mehrfach chelatierenden Liganden, ggf. geladen darstellen;
- L ein Mono- oder Bicarbenligand ist, der heterozyklisch ist; und
- A ein schwach koordinierendes Anion ist.

**3.** Katalytische Zusammensetzung nach Anspruch 2, **dadurch gekennzeichnet, dass** der Ligand L, einer der allgemeinen Formeln (I) und (II) der Beschreibung entspricht, in denen $R_1$, $R_2$, $R_3$, $R_4$, $R_5$ und $R_6$ identisch oder verschieden jedes Wasserstoff, eine Kohlenwasserstoffgruppe, aliphatisch, gesättigt oder ungesättigt oder aromatisch darstellen, die 1 bis 12 Kohlenstoffatome umfasst und Y einen bivalenten aliphatischen Rest mit 1 bis 4 Kohlenstoffatomen darstellt.

**4.** Katalytische Zusammensetzung nach einem der Ansprüche 2 oder 3, **dadurch gekennzeichnet, dass** L gewählt unter den Carbenliganden ist, die den Formeln (1), (2) und (3) der Beschreibung entsprechen.

**5.** Katalytische Zusammensetzung nach einem der Ansprüche 2 bis 4, **dadurch gekennzeichnet, dass** X und Y gewählt sind unter den Halogeniden, Carboxylaten, Acetylacetonat, Sulfat, Phenaten, Mono- und Diolefinen, $\pi$-Aromaten, den Alkylresten oder Arylresten, den Phosphinen, Phosphiten und Kohlenmonoxid.

**6.** Katalytische Zusammensetzung nach einem der Ansprüche 2 bis 5, **dadurch gekennzeichnet, dass** A gewählt ist unter den Anionen Tetrafluorborat, Hexafluorphosphat, Tetraphenylborat und Derivaten, Tetrachloraluminat, Hexafluorantimonat, Trifluoracetat, Trifluormethylsulfonat und Acetat.

**7.** Katalytische Zusammensetzung nach einem der Ansprüche 1 bis 6, **dadurch gekennzeichnet, dass** die Nickelverbindung gewählt wird unter

- $NiCl_2$, [1,3-Dimethylimidazol-2-yliden]$_2$;
- $NiI_2$ [1,3-Dimethylimidazol-2-yliden]$_2$;
- das Chlorid von $\pi$-Allylnickel (1,3-Dimethylimidazol-2-yliden);
- $NiI_2$ [1,1'-Dimethylimidazol-2,2'-Diyliden-3,3'-Methylen]$_2$; und
- $NiI_2$ [1,1'-Dimethylimidazol-2,2'-Diyliden-3,3'-Ethylen]$_2$.

**8.** Katalytische Zusammensetzung nach einem der Ansprüche 1 bis 7, **dadurch gekennzeichnet, dass** das quaternäre Ammoniumhalogenid oder quarternäre Phosphoniumhalogenid, das als Produkt A verwendbar ist, entspricht

- einer der allgemeinen Formeln:
  $NR^1R^2R^3R^4X$, mit Ausnahme von $NH_4X$, $PR^1R^2R^3R^4X$, $R^1R^2N{=}CR^3R^4X$ oder $R^1R^2P{=}CR^3R^4X$,

in denen X Cl oder Br darstellt und $R^1$, $R^2$, $R^3$ und $R^4$ identisch oder verschieden jedes Wasserstoff oder ein Kohlenwasserstoffrest mit 1 bis 12 Kohlenstoffatomen darstellen,
oder einer der allgemeinen Formeln

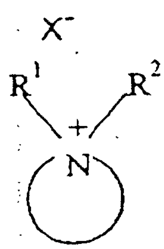 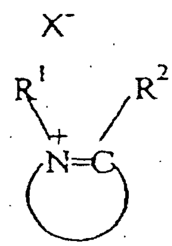 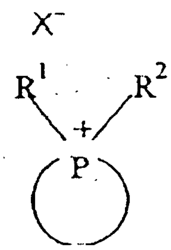 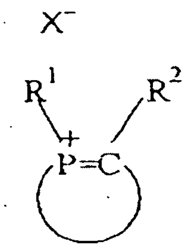

in denen die stickstoff- oder phosphorhaltigen Heterozyklen, die 1, 2 oder 3 Stickstoff- und/oder Phosphoratome umfassen, bestehen aus 4 bis 10 Atomen und X, $R^1$ und $R^2$, wie oben definiert sind.

**9.** Katalytische Zusammensetzung nach Anspruch 8, **dadurch gekennzeichnet, dass** das quarternäre Ammoniumhalogenid oder quarternäre Phosphoniumhalogenid das Chlorid von Tetrabutylphosphonium, das Chlorid von N-Butylpyridinium, das Bromid von Ethylpyridinium, das Chlorid von 3-Butyl-1-Methylimidazolium, das Chlorid von Diethylpyrazolium, das Chlorhydrat von Pyridinium oder das Chlorid von Trimethylphenylammonium, das Chlorid von 1-Ethyl-3-Methylimidazolium ist.

**10.** Katalytische Zusammensetzung nach einem der Ansprüche 1 bis 9, **dadurch gekennzeichnet, dass** das als Produkt B verwendete Aluminiumhalogenid das Trichlorid oder das Tribromid von Aluminium ist.

**11.** Katalytische Zusammensetzung nach einem der Ansprüche 1 bis 10, **dadurch gekennzeichnet, dass** die Produkte A und B in einem Molverhältnis A:B von 1:0,5 bis 1:3 eingesetzt werden.

**12.** Katalytische Zusammensetzung nach einem der Ansprüche 1 bis 11, **dadurch gekennzeichnet, dass** das nicht wässrige Milieu mit ionischem Charakter außerdem ein Produkt C umfasst, das aus wenigstens einer organischen Aluminiumverbindung besteht.

**13.** Katalytische Zusammensetzung nach Anspruch 12, **dadurch gekennzeichnet, dass** die organische, als Produkt C verwendete Aluminiumverbindung der allgemeinen Formel $AlR_xX_{3-x}$ entspricht, in der R ein linearer oder verzweigter Alkylrest ist, der 2 bis 8 Kohlenstoffatome umfasst, wobei X Chlor oder Brom ist und x einen Wert gleich 1, 2 oder 3 hat.

**14.** Katalytische Zusammensetzung nach Anspruch 12 oder 13, **dadurch gekennzeichnet, dass** das Produkt C, das Sesquichlorid von Isobutylaluminium, das Sesquichlorid von Ethylaluminium, das Dichlorisobutylaluminium, Dichlor-ethylaluminium oder Chlordiethylaluminium ist.

**15.** Katalytische Zusammensetzung nach einem der Ansprüche 12 bis 14, **dadurch gekennzeichnet, dass** das Produkt C in einem Molverhältnis mit dem Produkt B höchstens gleich 100:1 eingesetzt wird.

**16.** Verfahren zur Dimerisierung, Codimerisierung oder Oligomerisierung von wenigstens einem Olefin, **dadurch gekennzeichnet, dass** das Olefin mit einer katalytischen Zusammensetzung nach einem der Ansprüche 1 bis 15 kontaktiert wird.

**17.** Verfahren nach Anspruch 16, **dadurch gekennzeichnet, dass** die Reaktion zur Dimerisierung, Codimerisierung oder Oligomerisierung der Olefine im geschlossenen System, im halboffenen System oder kontinuierlich mit einer oder mehreren Reaktionsstufen unter Rühren bei einer Temperatur von -40 bis +70°C durchgeführt wird.

**18.** Verfahren nach einem der Ansprüche 16 und 17, **dadurch gekennzeichnet, dass** das (die) Olefin(e) gewählt ist (sind) unter Ethylen, Propylen, n-Butenen und n-Pentenen und deren Gemischen, rein oder verdünnt durch wenigstens ein Alkan.

**19.** Verfahren nach einem der Ansprüche 16 bis 18, **dadurch gekennzeichnet, dass** das (die) Olefin(e) in einer "Fraktion" aus einem Erdölraffinierungsverfahren enthalten ist (sind).

## Claims

**1.** Catalytic composition that results from the dissolution of at least one nickel compound that contains at least one heterocyclic mono- or bicarbene ligand in a medium that consists of

- at least one quaternary ammonium halide and/or at least one quaternary phosphonium halide, designated Product A;
- at least one aluminum halide, designated Product B;
- and optionally at least one organic aluminum compound, designated Product C.

**2.** Catalytic composition according to claim 1, in which said nickel compound corresponds to general formula $(Ni_aX_bY_dL_c)^n (A)_n$, in which

- a, b, c, d and n are integers with a equal to 1, 2 or 3; b equal to 0 to 2 x a; d equal to 0 to 2 x a; c equal to 1 to 4 x b; and n equal to 0, 1 or 2;
- X and Y, identical or different, each represent a mono- or multi-chelating ligand that may or may not be charged;
- L is a heterocyclic mono- or bi-carbene ligand; and
- A is a sparingly coordinating anion.

**3.** Catalytic composition according to claim 2, wherein ligand L corresponds to one of general formulas (I) and (II) of the description, in which $R_1$, $R_2$, $R_3$, $R_4$, $R_5$ and $R_6$, identical or different, each represent hydrogen, a hydrocarbon-containing group, aliphatic group, saturated or unsaturated group, or aromatic group, comprising 1 to 12 carbon atoms, and Y represents an aliphatic bivalent radical with 1 to 4 carbon atoms.

**4.** Catalytic composition according to claim 2 or 3, **characterized in that** L is selected from among the carbene ligands that correspond to formulas (1), (2) and (3) of the description.

**5.** Catalytic composition according to one of claims 2 to 4, wherein X and Y are selected from among the halides, carboxylates, acetylacetonate, sulfate, phenates, mono- and diolefins, π-aromatic compounds, alkyl or aryl radicals, phosphines, phosphites and carbon monoxide.

**6.** Catalytic composition according to one of claims 2 to 5, wherein A is selected from among the anions of tetrafluoroborate, hexafluorophosphate, tetraphenylborate and derivatives, tetrachloroaluminate, hexafluoroantimonate, trifluoroacetate, trifluoromethylsulfonate and acetate.

**7.** Catalytic composition according to claims 1 to 6, wherein the nickel compound is selected from among

- $NiCl_2$,[dimethyl-1,3-imidazolylidene-2]$_2$;
- $NiCl_2$,[dimethyl-1,3-imidazolylidene-2]$_2$;
- $\pi$-allyl nickel chloride (dimethyl-1,3-imidazolylidene-2);
- $NiCl_2$ [dimethyl-1,1'-imidazole-diylidene-2,2'-methylene-3,3']$_2$; and
- $NiCl_2$ [dimethyl-1,1'-imidazole-diylidene-2,2'-ethylene-3,3']$_2$.

**8.** Catalytic composition according to one of claims 1 to 7, wherein the quaternary ammonium halide or the quaternary phosphonium halide that can be used as Product A corresponds to:

- one of general formulas:
  $NR^1R^2R^3R^4X$, with the exception of $NH_4X$, $PR^1R^2R^3R^4X$, $R^1R^2N{=}CR^3R^4X$ or $R^1R^2P{=}CR^3R^4X$,
  in which X represents Cl or Br, and $R^1$, $R^2$, $R^3$ and $R^4$, identical or different, each represent hydrogen or a hydrocarbyl radical with 1 to 12 carbon atoms;
- or one of general formulas:

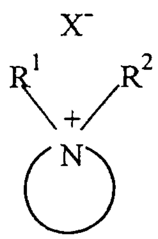
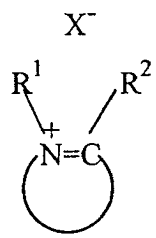
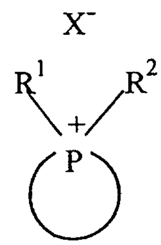
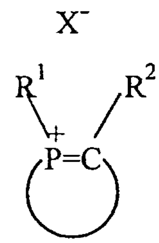

in which the nitrogen-containing heterocycles or phosphorus-containing heterocycles that comprise 1, 2 or 3 nitrogen atoms and/or phosphorus atoms consist of 4 to 10 atoms, and X, $R^1$ and $R^2$ are defined as above.

**9.** Catalytic composition according to claim 8, wherein the quaternary ammonium halide or quaternary phosphonium halide is tetrabutylphosphonium chloride, N-butylpyridinium chloride, ethylpyridinium bromide, butyl-3 methyl-1 imidazolium chloride, diethylpyrazolium chloride, pyridinium chlorohydrate, trimethylphenylammonium chloride or ethyl-1 methyl-3 imidazolium chloride.

**10.** Catalytic composition according to one of claims 1 to 9, wherein the aluminum halide that is used as Product B is aluminum trichloride or aluminum tribromide.

**11.** Catalytic composition according to one of claims 1 to 10, wherein Products A and B are used in a molar ratio A:B of 1:0.5 to 1:3.

**12.** Catalytic composition according to one of claims 1 to 11, wherein the non-aqueous ionic medium also comprises a Product C that consists of at least one organic aluminum compound.

**13.** Catalytic composition according to claim 12, wherein the organic aluminum compound that is used as Product C corresponds to general formula $AlR_xX_{3-x}$, in which R is an alkyl, linear or branched radical, comprising 2 to 8 carbon atoms, whereby X is chlorine or bromine and x has a value that is equal to 1, 2 or 3.

**14.** Catalytic composition according to claim 12 or 13, wherein Product C is isobutylaluminum sesquichloride, ethylaluminum sesquichloride, dichloroisobutylaluminum, dichloroethylaluminum or chlorodiethylaluminum.

**15.** Catalytic composition according to one of claims 12 to 14, wherein Product C is used in a molar ratio with Product B that is at most equal to 100:1.

**16.** Process of dimerization, codimerization or oligomerization of at least one olefin, wherein said olefin is brought into contact with a catalytic composition according to one of claims 1 to 15.

17. Process according to claim 16, wherein the reaction of dimerization, codimerization or oligomerization of the olefins is conducted in a closed system, semi-open system or continuously with one or more reaction stages, while being stirred and at a temperature of -40 to +70°C.

18. Process according to one of claims 16 and 17, wherein the olefin(s) is (are) selected from among ethylene, propylene, n-butenes and n-pentenes and mixtures thereof that are pure or diluted by at least one alkane.

19. Process according to one of claims 16 to 18, wherein the olefin(s) is (are) contained in a "fraction" that is obtained from a petroleum refining process.